# EUROPÄISCHE PATENTSCHRIFT

(11) **EP 3 106 450 B1**
(45) Veröffentlichungstag und Bekanntmachung des Hinweises auf die Patenterteilung: **25.07.2018**
(21) Anmeldenummer: 15400024.4
(22) Anmeldetag: 19.06.2015
(51) Int. Cl.: C07C 11/06, C07C 1/20, C07C 7/04, C07C 7/08, C07C 2/66, C07C 15/02, C07C 5/10, C07C 13/18, C10G 3/00, C10G 45/44, C10G 69/12, C10G 7/00, C07C 41/09, C07C 43/04, C10G 29/20

(54) **VERFAHREN UND ANLAGE ZUR RÜCKGEWINNUNG UND NUTZUNG HÖHERER OLEFINE BEI DER OLEFINSYNTHESE AUS OXYGENATEN**
METHOD AND INSTALLATION FOR RECOVERING AND USING HIGHER OLEFINS IN OLEFIN SYNTHESIS FROM OXYGENATES
PROCEDE ET INSTALLATION DE RECUPERATION ET D'UTILISATION D'OLEFINES SUPERIEURES LORS DE LA SYNTHESE D'OLEFINES A PARTIR DE PRODUITS OXYGENES

(43) Veröffentlichungstag der Anmeldung: 21.12.2016
(73) Patentinhaber: L'AIR LIQUIDE, SOCIETE ANONYME POUR L'ETUDE ET L'EXPLOITATION DES PROCEDES GEORGES CLAUDE, 75007 Paris (FR)
(72) Erfinder: Bauer, Ingo, 61118 Bad Vilbel (DE); Rothämel, Martin, 60437 Frankfurt am Main (DE)
(74) Vertreter: Dropsch, Holger

(56) Entgegenhaltungen:
- WO-A1-2014/124844
- CN-A- 103 772 109
- DE-A1-102013 101 578

## Beschreibung

### Gebiet der Erfindung

Die vorliegende Erfindung betrifft ein Verfahren zur Herstellung von Olefinen aus Oxygenaten mit folgenden Schritten:
(a) heterogen-katalysierte Umsetzung eines Wasserdampf und Oxygenate, wie Methanol und/oder Dimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trenriverfahren in eine C₅₋-Fraktion, eine C₅₊-Fraktion und eine wässrige, nicht umgesetzte Oxygenate enthaltende Phase,
(c) Auftrennung der C₅₋-Fraktion durch mehrstufige Destillation, wobei ein Propylenproduktstrom und mehrere olefinhaltige Kohlenwasserstofffraktionen erhalten werden, wobei letztere mindestens teilweise zum Olefinsynthesereaktor zurückgeführt werden,
(d) Abtrennung der nicht umgesetzten Oxygenate aus der wässrigen Phase mittels eines thermischen Trennverfahrens, Rückführung der abgetrennten, nicht umgesetzten Oxygenate und mindestens eines Teils des Wassers zum Olefinsynthesereaktor,
(e) Auftrennung der C₅₊-Fraktion durch mehrstufige Destillation, wobei eine C₇₋-Fraktion erhalten wird, die C₅/C₆-Olefine umfasst, und wobei eine C₇₊-Fraktion erhalten wird, die Aromaten umfasst.

Weiterhin umfasst die Erfindung eine Anlage zur Durchführung dieses Verfahrens.

### Stand der Technik

Propen (C₃H₆), oft auch als Propylen bezeichnet, ist einer der wichtigsten Ausgangsstoffe der chemischen Industrie. Die Nachfrage nach dem Grundstoff Propylen steigt weltweit, wobei Propylen ebenso wie Ethylen zumeist in einem Steam-Cracker in einem von den Verfahrensbedingungen und den Rohstoffen abhängigen Verhältnis aus Erdöl erzeugt wird.

Um zusätzliches Propylen zu gewinnen, existiert eine Reihe von Verfahren, wie etwa die Propandehydrierung, die von Propan als Edukt ausgeht. Bekannt ist jedoch vor allem das so genannte MTP-(Methanol-to-Propylene)-Verfahren, in dem sogenannte Oxygenate wie Methanol (MeOH) oder Dimethylether (DME) durch katalytische Umsetzung an einem zeolithischen Katalysator zu Olefinen umgewandelt werden. Durch Variation des Katalysators und der Prozessbedingungen kann die Selektivität der erhaltenen Produkte beeinflusst und so das Produktspektrum zu kurzkettigen Olefinen (dann oft auch die Prozessbezeichnung Methanol-to-Olefin (MTO)), zu längerkettigen Produkten (dann oft auch die Prozessbezeichnung Methanol-to-Gasoline (MTG)) oder eben zu Propylen verschoben werden.

Die Grundlagen eines MTP-Verfahrens sind beispielsweise in der Offenlegungsschrift DE 10 2005 048 931 A1 beschrieben. Aus einem Wasserdampf und Oxygenate, wie Methanol und/oder Dimethylether, enthaltenden Eduktgemisch werden vor allem C₂- bis C₄-Olefine hergestellt. Dabei wird das Eduktgemisch in wenigstens einem Reaktor durch eine heterogen-katalysierte Reaktion zu einem niedermolekulare Olefine und Benzinkohlenwasserstoffe umfassenden Reaktionsgemisch umgesetzt. Durch ein geeignetes Trennkonzept können höherwertige Olefine, vor allem die C₅₊-Fraktion, als Recyclingstrom wenigstens teilweise in den Reaktor zurückgeführt und dort größtenteils zu Propylen umgewandelt werden, wodurch sich die Ausbeute an Propylen erhöht.

Das MTP-Verfahren hat üblicherweise eine Propylenausbeute von etwa 65% (mol-C). Eine erhöhte Propylenausbeute würde die Wirtschaftlichkeit des Verfahrens deutlich verbessern. Als vorherrschendes Nebenprodukt im MTP-Prozess fällt ein benzinartiges Gemisch (MTP-Benzin) an, das im Wesentlichen aus Olefinen, Paraffinen, cyclischen Kohlenwasserstoffen und Aromaten besteht. Dieses MTP-Benzin kann ebenfalls in eine sich anschließende Wertschöpfungskette eingegliedert werden, hat jedoch einen niedrigeren Marktpreis als Propylen.

Wie in der Druckschrift WO 2006/136433 A1 beschrieben, wird daher beispielsweise versucht, das MTP-Benzin bzw. Fraktionen von diesem einer Nachbearbeitung in Form einer Olefin-Metathese zu unterziehen, bei der das MTP-Benzin bzw. aus diesem gewonnene Fraktionen bei Temperaturen von etwa 400 bis 500 °C und einem Druck von 1 bis 5 bar an einem zeolithischen Katalysator umgesetzt wird. Durch diese nachgeschaltete Reaktion kann zwar eine moderate Erhöhung der Propylen-Ausbeute des Gesamtverfahrens erreicht werden, allerdings liegt die Gesamtausbeute immer noch unter 70 mol-%.

Eine direkte Rückführung des MTP-Benzins in den MTP-Reaktor bringt keine Steigerung der Ausbeute an Propylen. Da es innerhalb des MTP-Reaktors zu unerwünschten Alkylierungsreaktionen der Aromaten kommt, die Methanol verbrauchen, welches dann nicht mehr für die selektive Bildung von Propylen zur Verfügung steht, sänke die Propylenausbeute des Gesamtverfahrens bei der direkten Rückführung des MTP-Benzins in den MTP-Reaktor sogar.

Einige Verfahren stellen daher darauf ab, die erhaltenen schwereren Olefine so umzuwandeln, dass zumindest ein Produkt mit homogener Zusammensetzung und höherem Markpreis entsteht. So lehrt beispielsweise die Patentschrift US 4,543,435, dass wenigstens ein Teil der erhaltenen Olefine zu schweren Kohlenwasserstoffen umgesetzt werden soll, so dass die Ausbeute an Flüssiggas und Benzin innerhalb des MTP-Verfahrens gesteigert werden kann.

Die WO 2011/131647 beschreibt ein Verfahren zur Herstellung von aromatischen Kohlenwasserstoffen, bei denen ein Feed von leichten Alkanen an einem geeigneten Katalysator wenigstens teilweise zu Aromaten umgesetzt wird. Parallel dazu läuft ein MTO-Verfahren ab. Dabei wird ein Teil des Oxygenat-Feeds des MTO-Verfahrens dadurch hergestellt, dass der bei der Umsetzung der Alkane zu Aromaten entstehende Wasserstoff mit Kohlenmonoxid und/oder Kohlendioxid zu einem Oxygenat umgewandelt wird. Die so entstehenden Produktströme lassen sich leicht mit den anderen Nebenprodukten des MTP-Prozesses, vornehmlich Methan, Kohlenoxiden, Wasserstoff und einem flüssiggasähnlichen Produkt, zusammenführen.

Zur Erhöhung der Ausbeute an werthaltigen Produkten aus einem MTP-Verfahren ist auch eine Hydrierung der erhaltenen aromatischen Kohlenwasserstoffe bekannt. So lehrt die US 2004/0039239, dass höherwertige Olefine an einem geeigneten Hydrierungskatalysator hydriert werden sollen. Insbesondere dadurch, dass auch Aromaten zu Paraffinen hydriert werden, kann so die Ausbeute eines benzinartigen Wertproduktes gesteigert werden.

Aus der US-Patentschrift US 4,482,772 ist eine Hydrierung innerhalb eines MTP-Verfahrens bekannt, bei dem zuerst die Umsetzung der Oxygenate zu Olefinen abläuft und die so erhaltenen Olefine anschließend oligomerisiert werden. Im Anschluss an die Oligomerisierung werden zumindest Teile des Produktstroms hydriert, wodurch im Produktstrom enthaltene Aromaten zu Cycloparaffinen umgewandelt werden. Damit lässt sich ebenfalls die Ausbeute eines benzinartigen Wertproduktes steigern.

Zur Durchführung solcher Hydrierungen sind beispielsweise aus der Patentanmeldung US 2007/0284284 A1 verschiedene Katalysatorarten und deren Anwendungsmöglichkeiten bekannt. Die Druckschrift DE 10 2013 101 578 A1 beschreibt ein Verfahren zur Herstellung von Olefinen aus Oxygenaten mit folgenden Schritten: (i) heterogen-katalysierte Umsetzung von wenigstens einem Oxygenat zu einem Propylen, Aromaten und zyklische Olefine enthaltenden Strom und (ii) Olefin-Interkonversion von wenigstens einem Teilstrom des Propylen, Aromaten und zyklische Olefine enthaltenden Stroms. Ziel ist es, ausgehend von Oxygenaten die Propylenausbeute deutlich zu erhöhen. Schließlich schlägt die internationale Patentanmeldung WO 2014/124844 A1 ein modifiziertes MTP-Verfahren vor, bei dem eine Aromatenfraktion gewonnen und diese durch Hydrierung in eine Cycloparaffine enthaltende Fraktion umgewandelt wird. Letztere wird als Recyclestrom zu dem Olefinsynthesereaktor zurückgeführt und in diesem teilweise zu Olefinen, darunter Propylen, umgesetzt. Auf diese Weise wird bereits eine Steigerung der Propylenausbeute erreicht, allerdings bleiben immer noch Reaktivkomponenten, insbesondere C₅₊-Olefine, für die Propylenbildung ungenutzt und werden beispielweise dem MTP-Benzin zugeschlagen. Ein anderer Teil der C₅₊-Olefine wird über Spülströme dem Verfahren entzogen, deren eigentliche Aufgabe es ist, eine Anreicherung der C₅₊-Paraffine im MTP-Verfahren zu verhindern.

### Beschreibung der Erfindung

Vor diesem Hintergrund besteht die Aufgabe der vorliegenden Erfindung darin, ein Verfahren bereitzustellen, bei dem durch verbesserte Nutzung und Rückführung der Nebenprodukte der Olefinsynthesereaktion aus Oxygenaten, insbesondere der C₅₊-Olefine und der Aromaten, die Propylenausbeute deutlich vergrößert werden kann.

Diese Aufgabe wird durch ein Verfahren mit den Merkmalen des Anspruchs 1 und durch eine Anlage mit den Merkmalen des Anspruchs 10 gelöst.

### Erfindungsgemäßes Verfahren:

Verfahren zur Herstellung von Olefinen, insbesondere Propylen, aus Oxygenaten, umfassend die folgenden Schritte:
(a) heterogen-katalysierte Umsetzung eines Wasserdampf und Oxygenate, wie Methanol und/oder Dimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
(c) Auftrennung der C₅₋-Fraktion durch mehrstufige Destillation, wobei ein Propylenproduktstrom und mehrere olefinhaltige Kohlenwasserstofffraktionen erhalten werden, wobei letztere mindestens teilweise zum Olefinsynthesereaktor zurückgeführt werden,
(d) Abtrennung der nicht umgesetzten Oxygenate aus der wässrigen Phase mittels eines thermischen Trennverfahrens, Rückführung der abgetrennten, nicht umgesetzten Oxygenate und mindestens eines Teils des Wassers zum Olefinsynthesereaktor,
(e) Auftrennung der C₅₊-Fraktion durch ein thermisches Trennverfahren, wobei eine C₇₋-Fraktion erhalten wird, die C_{5/}C₆-Olefine umfasst, und wobei eine C₇₊-Fraktion erhalten wird, die Aromaten umfasst,
(f) Auftrennung der C₇₊-Fraktion durch ein thermisches Trennverfahren, bevorzugt durch Extraktivdestillation, in eine Aromatenfraktion und eine die C₇₊-Paraffine umfassende Fraktion,
(g) Zuführung mindestens eines Teils der die C₅/C₆-Olefine umfassenden C₇₋-Fraktion und mindestens eines Teils der Aromatenfraktion zu einem Alkylierungsreaktor, in dem durch heterogen-katalysierte Umsetzung der Aromaten mit den C₅/C₆-Olefinen an einem Alkylierungskatalysator unter Alkylierungsbedingungen ein Alkylierungsprodukt erhalten wird, das Alkylaromaten umfasst,
(h) Auftrennung des Alkylierungsprodukts durch ein thermisches Trennverfahren in einen C₅/C₆-Paraffine enthaltenden Strom, einen Alkylaromatenstrom und einen nicht umgesetzte Aromaten enthaltenden Strom, der mindestens teilweise zum Alkylierungsreaktor zurückgeführt wird,
(i) Zuführung des Alkylaromatenstroms zu einem Hydrierungsreaktor, in dem durch heterogen-katalysierte Umsetzung der Alkylaromaten an einem Hydrierungskatalysator unter Hydrierungsbedingungen ein Hydrierungsprodukt erhalten wird, das Cycloparaffine umfasst,
(j) Rückführung mindestens eines Teils des Hydrierungsprodukts zum Olefinsynthesereaktor.

### Erfindungsgemäße Anlage:

Anlage zur Herstellung von Olefinen aus Oxygenaten, insbesondere zur Durchführung des Verfahrens nach einem der vorhergehenden Ansprüche, umfassend folgende miteinander in Fluidverbindung stehenden Anlagenteile:
- wenigstens einen Olefinsynthesereaktor zur heterogen-katalysierten Umsetzung von wenigstens einem Oxygenat zu Propylen, andere Olefine, Paraffine und Aromaten enthaltenden Primärprodukt,
- eine Trennvorrichtung zur Auftrennung des Primärprodukts in eine C₅₋-Fraktion, eine C₅₊-Fraktion und eine wässrige, nicht umgesetzte Oxygenate enthaltende Phase,
- eine Trennvorrichtung zur Auftrennung der C₅₋-Fraktion in einen Propylenproduktstrom und mehrere olefinhaltige Kohlenwasserstofffraktionen, sowie Rückführleitungen zur Rückführung mindestens eines Teils der olefinhaltigen Kohlenwasserstofffraktionen zum Olefinsynthesereaktor,
- eine Trennvorrichtung zur Abtrennung der nicht umgesetzten Oxygenate aus der wässrigen Phase, sowie Rückführleitungen zur Rückführung mindestens eines Teils der nicht umgesetzten Oxygenate und mindestens eines Teils des Wassers zum Olefinsynthesereaktor,
- eine Trennvorrichtung zur Auftrennung der C₅₊-Fraktion in eine C₇₋-Fraktion, die C₅/C₆-Olefine umfasst, und in eine C₇₊-Fraktion, die Aromaten umfasst,
- eine Trennvorrichtung zur Auftrennung der C₇₊-Fraktion in eine Aromatenfraktion und eine die C₇₊-Paraffine umfassende Fraktion,
- einen Alkylierungsreaktor zur heterogen-katalysierten Umsetzung von Aromaten mit C₅/C₆-Olefinen zu einem Alkylierungsprodukt, das Alkylaromaten umfasst, sowie Leitungen für die Zuführung mindestens eines Teils der die C₅/C₆-Olefine umfassenden C₇₋-Fraktion und mindestens eines Teils der Aromatenfraktion zu dem Alkylierungsreaktor,
- eine Trennvorrichtung zur Auftrennung des Alkylierungsprodukts in einen C₅/C₆-Paraffine enthaltenden Strom, einen Alkylaromatenstrom und einen nicht umgesetzte Aromaten enthaltenden Strom, sowie eine Rückführleitung für die Rückführung mindestens eines Teils des nicht umgesetzte Aromaten enthaltenden Stroms zum Alkylierungsreaktor,
- einen Hydrierungsreaktor zur Hydrierung der Alkylaromaten zu einem Hydrierungsprodukt, das Cycloparaffine umfasst,
- eine Rückführleitung zur Rückführung mindestens eines Teils des Hydrierungsprodukts zum Olefinsynthesereaktor.

Die für die Umsetzung von Oxygenaten zu Olefinprodukten erforderlichen Oxygenatumsetzungsbedingungen, die für die Alkylierung von Aromaten mit Olefinen erforderlichen Alkylierungsbedingungen und die für die Hydrierung von Alkylaromaten zu Cycloparaffinen erforderlichen Hydrierungsbedingungen sind dem Fachmann aus dem Stand der Technik, beispielsweise den eingangs erörterten Druckschriften, bekannt. Notwendige Anpassungen dieser Bedingungen an die jeweiligen Betriebserfordernisse wird er auf der Grundlage von Routineversuchen vornehmen. Dabei können die nachfolgend offenbarten, spezifischen Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind die die Destillation oder Rektifikation. Grundsätzlich ist aber auch der Einsatz anderer thermischer Trennverfahren denkbar, beispielsweise der Extraktion oder Extraktivdestillation.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Zur Bezeichnung von Kohlenwasserstofffraktionen wird folgende Nomenklatur verwendet: "Cₙ-Fraktion" bezeichnet eine Kohlenwasserstofffraktion, die hauptsächlich Kohlenwasserstoffe der C-Kettenlänge n, also mit n C-Atomen, enthält. "Cₙ₋-Fraktion" bezeichnet eine Kohlenwasserstofffraktion, die Kohlenwasserstoffe der C-Kettenlänge n sowie mit niedrigeren C-Kettenlängen enthält. "Cₙ₊-Fraktion" bezeichnet eine Kohlenwasserstofffraktion, die Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cₙ₋-Fraktion je nach Verfahrensbedingungen des Trennverfahrens immer noch geringe Mengen von Kohlenwasserstoffen mit einer C-Zahl größer als n.

Der Erfindung liegt die Erkenntnis zugrunde, dass C₅/C₆-Olefine auf effiziente und selektive Weise aus der C₇₋-Fraktion abgetrennt werden können, indem sie mit einer verfahrenseigenen Aromatenfraktion zu Alkylaromaten umgesetzt werden. Dies hat den Vorteil, dass dem Gesamtverfahren keine Olefine verloren gehen, die durch Rückführung zumindest teilweise zum Zielprodukt Propylen umgesetzt werden können.

In einem nachfolgenden Schritt werden die erhaltenen Alkylaromaten zu Cycloparaffinen, im Wesentlichen zu Cyclohexanderivaten mit Alkyl-Seitenkette, hydriert. Die Hydrierung ist notwendig, da ansonsten die Aromaten bei Rückführung zum Olefinsynthesereaktor in unerwünschter Weise durch Methanol alkyliert werden würden. Die somit erhaltenen Cycloparaffine können dann in die heterogen-katalysierte Olefinsynthese zurückgeführt werden, wo sie sich in bekannter Weise zu Olefinen mit einer geringeren Kettenlänge, insbesondere zu Propylen, umsetzen, ohne dass unerwünschte Alkylierungsreaktionen auftreten, da die Cycloparaffine von ihrer chemischen Summenformel her (CₙH₂ₙ) schon exakt Olefinen entsprechen. Die Umsetzung erfolgt nach Ringöffnung mit moderatem Umsatz, aber guter Selektivität zu Ethylen, Propylen und höheren Olefinen.

### Bevorzugte Ausgestaltungen der Erfindung

Vorteilhafterweise wird für die Hydrierreaktion Wasserstoff (H₂) als Hydrierungsmittel eingesetzt. Da die Aromaten und cyclischen Olefine aus der MTP-Reaktion stammen, weisen sie keinerlei anorganische Katalysatorgifte wie Schwefel etc. auf. Somit können zur Hydrierung sehr moderate Bedingungen bei Temperaturen von weniger als 150 °C und Drücken von weniger als 25 bar verwendet werden, um einen nahezu vollständigen Umsatz zu erreichen. Es können Standard-Hydrierkatalysatoren verwendet werden, die z.B. Nickel oder Palladium als Aktivkomponente enthalten, die auf einem Träger, z. B. Aktivkohle, aufgebracht ist.

Es hat sich überdies als vorteilhaft herausgestellt, die Hydrierung so durchzuführen, dass Teile des Produktstroms der Hydrierung in die Hydrierung zurückgeführt werden und dass das Verhältnis zwischen dem zurückgeführten hydrierten Produkt und nicht-hydrierten Alkylaromaten zwischen 1 kg/kg und 10 kg/kg liegt. Durch die Einstellung dieses Verhältnisses kann eine Verdünnung der umzusetzenden Edukte erreicht werden. Dies ist deshalb notwendig, weil die Hydrierung eine stark exotherme Reaktion ist und anderenfalls innerhalb der Hydrierung eine zu starke Aufwärmung des Reaktionsgemisches erfolgt. Die Verwendung von hydriertem Produkt als Verdünnungsmittel hat den Vorteil, dass so keine weiteren Komponenten in das Verfahren eingebracht werden. Bei dem hydrierten Produkt ist zwischen flüssigen Produktströmen und gasförmigen Produktströmen, welche im Wesentlichen aus nicht umgesetztem Wasserstoff und gebildeten leichten Gasen bestehen, zu unterscheiden. Aufgrund der größeren Dichte ist es vorteilhaft, das Flüssigprodukt zur Verdünnung der Hydrierung zu verwenden.

Der molare Überschuss von Wasserstoff sollte zwischen 200 und 5000 % der theoretisch für die vollständige Absättigung aller vorhandenen Doppel- und aromatischen Bindungen notwendigen Menge liegen. So kann eine Limitierung der Reaktion aufgrund begrenzter lokaler Wasserstoffkonzentrationen vollständig ausgeschlossen werden.

Indem nach der Hydrierung Wasserstoff abgetrennt wird, ist der Überschuss an Wasserstoff für die weitere Verarbeitung des hydrierten Stroms unerheblich, zudem kann der nicht umgesetzte Wasserstoff wieder in die Hydrierung zurückgeführt werden, so dass es zu keinem tatsächlich höheren Wasserstoffverbrauch kommt. Dementsprechend muss nur die stöchiometrisch notwendige Menge an Wasserstoff stetig dazugegeben werden. Eine Anreicherung von gasförmigen Nebenprodukten der Hydrierung wie z.B. Methan kann dadurch kontrolliert werden, dass man einen stetigen kleinen Purgestrom aus dem Prozessteil entfernt.

Weiterhin hat es sich als vorteilhaft herausgestellt, bereits nach der heterogenkatalysierten Olefinsynthese eine C₅-Fraktion vom verbleibenden Reststrom abzutrennen. So können die besonders werthaltigen niedermolekularen Olefine, insbesondere das Propylen selbst, sofort aus dem Produktstrom der heterogen-katalysierten Umsetzung abgezogen werden, weshalb alle sich anschließenden Anlagenkomponenten für die Aufarbeitung der höherwertigen Olefine kleiner dimensioniert werden können.

Die Abtrennung erfolgt vorteilhafterweise durch eine Abkühlung, bei der die C₅-Fraktion gasförmig bleibt und sich aufgrund ihres Aggregatzustandes von dem verbleibenden flüssigen Reststrom trennt. Die so gewonnene Energie kann an anderer Stelle im Prozess genutzt werden.

Aus dem verbleibenden Reststrom wird zum einen eine wässrige, Oxygenate enthaltene Fraktion und zum anderen eine C₅₊-Fraktion gewonnen.. Vorzugsweise wird dies durch eine einfache Phasentrennung erreicht, wodurch hohe Energiekosten, wie etwa eine Destillation sie bedingt, entfallen.

Vorteilhafterweise wird die wässrige, Oxygenate enthaltende Fraktion einer Trennoperation unterzogen, bei der die Oxygenate, also im Wesentlichen Methanol und/oder Dimethylether und das Wasser voneinander getrennt werden. Diese Trennung erfolgt bevorzugt in einer Destillation, um eine ausreichende Trennschärfe sicherzustellen. Wenigstens Teile des Wassers werden aus dem Verfahren ausgeschleust. Abgetrenntes Methanol und/oder abgetrenntes Wasser können in die heterogene-katalysierte Olefinsynthese zurückgeführt werden. Die Rückführung des Wassers erfolgt vorzugsweise als Dampf.

In einer besonders bevorzugten Ausgestaltung des Gesamtverfahrens erfolgt die Olefinsynthese in zwei Stufen, wobei in der ersten Stufe das wenigstens eine Oxygenat zuerst zu wenigstens einem korrespondierenden Ether und in der zweiten Stufe der/die Ether zu Olefinen umgesetzt wird/werden. Wird Methanol als Oxygenat verwendet, folgt zuerst eine Umsetzung des Methanols zu Dimethylether und anschließend die Umsetzung des Dimethylethers zu Propylen und anderen Olefinen, insbesondere auch zu Aromaten. Bei dieser zweistufigen Ausgestaltung empfiehlt es sich, Methanol bereits vor die erste Stufe, also vor die Umsetzung zu Dimethylether, zurückzuführen, während das dampfförmige Wasser zwischen die erste und zweite Stufe eingebracht wird, da es erst für die Umsetzung des Ethers zu Olefinen als Edukt verwendet werden muss. Somit wird in der ersten Stufe kein unnötiges Wasser eingesetzt, welches die Gleichgewichtsreaktion bei der Veretherung negativ beeinflusst; der Dampf steht jedoch als Edukt in der Olefinsynthese zur Verfügung.

Das Produkt aus der Oxygenatumsetzungsreaktion wird mittels dem Fachmann bekannten Methoden zunächst gekühlt, wobei Wasser und im Wasser lösliche Komponenten wie Oxygenate (Methanol, DME) auskondensiert werden und damit auf einfache Weise vom restlichen Kohlenwasserstoffprodukt abgetrennt werden können. Der resultierende wässrige Strom wird dann einer geeigneten Trenneinrichtung (z.B. einer Destillationskolonne) zugeführt, wobei die Oxygenate wie oben beschrieben in die erste Reaktionsstufe zurückgeführt werden. Die aus der Umsetzung der Oxygenate entstandene Wassermenge wird aus dem Prozess entfernt, während die Restmenge wie oben beschrieben vor die zweite Reaktionsstufe zurückgeführt wird, so dass sich größtenteils geschlossene Kreisläufe ergeben.

Der weitgehend wasserfreie Kohlenwasserstoffstrom nach der Kühlung wird komprimiert; es entsteht ein unter Druck stehender leichter Kohlenwasserstrom und eine flüssiger, ebenfalls unter Druck stehender schwerer Kohlenwasserstoffstrom. Um eventuell noch enthaltene leichtere Olefine sicher abzutrennen, empfiehlt es sich, eine weitere Trennstufe zu schalten, in der aus der C₅₊-Fraktion noch eventuell enthaltene C₄₋-Fraktionen entfernt werden können. Vorteilhafterweise ist diese Trennstufe eine Destillation, um eine ausreichende Trennschärfe sicherzustellen.

Bevorzugt werden zumindest Teile der die C₅/C₆-Olefine umfassenden C₇₋-Fraktion zum Olefinsynthesereaktor zurückgeführt. Dadurch, dass nicht die gesamte C₇₋-Fraktion zu der Alkylierung geführt wird, kann der Alkylierungsreaktor und der nachfolgende Hydrierungsreaktor kleiner ausgestaltet werden. Es wird somit ein Kompromiss zwischen geringeren Investitionskosten, bedingt durch die kleineren Apparategrößen, und einem moderaten Verlust an C₅/C₆-Olefinen erreicht.

Weiterhin hat es als vorteilhaft herausgestellt, wenn die Abtrennung der C₇₋- von der C₇₊-Fraktion aus der zuvor abgetrennten C₅₊-Fraktion erfolgt. Dies hat den Vorteil, dass durch die frühzeitige Abtrennung der C₅-Fraktion die geführten Ströme deutlich kleiner sind und somit der apparative Aufwand geringer wird.

In einer Weiterbildung der Erfindung empfiehlt es sich zudem, mindestens einen Teil des in Verfahrensschritt (h) erhaltenen, nicht umgesetzte Aromaten enthaltenden Stroms dem Hydrierungsreaktor zuzuführen. Dadurch kann die Ausbeute an Cycloparaffinen weiter gesteigert und somit der Recycle an umwandlungsfähigem Material zum Olefinsynthesereaktor erhöht werden.

Als besonders vorteilhaft hat es sich herausgestellt, dass in dem Olefinsynthesereaktor und in dem Alkylierungsreaktor derselbe Katalysator verwendet wird. Außer logistischen Vorteilen besitzt dies den Vorzug, dass bei der Alkylierung weitgehend nur Alkylierungsprodukte gebildet werden, die klein genug sind, um das Porensystem des Katalysators zu verlassen. Dieser Vorzug ist insbesondere bei Verwendung zeolithbasierter Katalysatoren relevant. Da sich die Molekülgröße durch die nachfolgende Hydrierung nur unwesentlich ändert, können die Alkylierungsprodukte somit auch in den im Olefinsynthesereaktor eingesetzten Katalysator eindiffundieren und dort umgesetzt werden.

In vorteilhafter Weise liegt im Verfahrensschritt (g) das Verhältnis der Summe der Mole von Aromaten in den aromatenhaltigen Strömen bzw. Fraktionen zur Summe der Mole an Olefinen in der dem Alkylierungsreaktor zugeführten C₇₋-Fraktion zwischen 1 und 10 mol/mol, bevorzugt zwischen 1 und 5 mol/mol, meist bevorzugt zwischen 1 und 2 mol/mol, so dass sichergestellt ist, dass ein vollständiger Umsatz der Olefine durch Alkylierung mit einem Aromaten-Molekül stöchiometrisch möglich ist. Ein Überschuss der Summe der Aromaten zu der Summe der Olefine im Feed zum Alkylierungsreaktor stellt zudem sicher, dass unerwünschte Mehrfachalkylierungen nur in untergeordnetem Maße auftreten. Diese unerwünschten Mehrfachalkylierungen werden allerdings auch durch die Porenstruktur der verwendeten Katalysatoren (z.B. ZSM-5) minimiert, da größere Moleküle innerhalb der Porenstruktur gar nicht gebildet werden können.

In besonderer Ausgestaltung der erfindungsgemäßen Anlage weist diese eine Rückführleitung für die Rückführung mindestens eines Teils der die C₅/C₆-Olefine umfassenden C₇₋-Fraktion zum Olefinsynthesereaktor auf. Dadurch, dass nicht die gesamte C₇₋-Fraktion zu der Alkylierung geführt wird, kann der Alkylierungsreaktor und der nachfolgende Hydrierungsreaktor kleiner ausgestaltet werden. Es wird somit ein Kompromiss zwischen geringeren Investitionskosten, bedingt durch die kleineren Apparategrößen, und einem moderaten Verlust an C₅/C₆-Olefinen erreicht.

Vorteilhaft ist es ferner, wenn die Anlage auch eine Leitung zur Zuführung mindestens eines Teils des die nicht umgesetzten Aromaten enthaltenden Stroms zum Hydrierungsreaktor umfasst. Durch die Zuführung eines Teils der nicht umgesetzter Aromaten zum Hydrierungsreaktor kann die Ausbeute an Cycloparaffinen weiter gesteigert und somit der Recycle an umwandlungsfähigem Material zum Olefinsynthesereaktor erhöht werden.

In einem weiteren Aspekt der Erfindung umfasst die Anlage auch Rückführleitungen für die Rückführung mindestens eines Teils des nicht umgesetzten Wasserstoffs und mindestens eines Teils des Hydrierungsprodukts zum Hydrierungsreaktor. Durch die Rückführung des Hydrierungsprodukts kann eine Verdünnung der umzusetzenden Edukte erreicht werden. Dies ist deshalb notwendig, weil die Hydrierung eine stark exotherme Reaktion ist und anderenfalls innerhalb der Hydrierung eine zu starke Aufwärmung des Reaktionsgemisches erfolgt. Die Verwendung von hydriertem Produkt als Verdünnungsmittel hat den Vorteil, dass so keine weiteren Komponenten in das Verfahren eingebracht werden. Durch die Rückführung nicht umgesetzten Wasserstoffs kann der erwünschte, hohe Wasserstoffüberschuss im Hydrierungsreaktor erreicht werden, wobei nur der tatsächlich stöchiometrisch verbrauchte Wasserstoff als Feed neu zugeführt werden muss. So kann eine Limitierung der Reaktion aufgrund begrenzter lokaler Wasserstoffkonzentrationen vollständig ausgeschlossen werden.

### Ausführungs- und Zahlenbeispiel

Weitere Merkmale, Vorteile und Anwendungsmöglichkeiten der Erfindung ergeben sich auch aus der nachfolgenden Beschreibung von Ausführungs- und Zahlenbeispielen und der Zeichnungen. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung.

Es zeigen:
- Fig. 1: die schematische Darstellung eines MTP-Verfahrens und einer entsprechenden Anlage gemäß Stand der Technik,
- Fig. 2.: die schematische Darstellung des erfindungsgemäßen Verfahrens bzw. der erfindungsgemäßen Anlage.

Der nachfolgend verwendete Begriff "Kolonne" bezieht sich grundsätzlich auf eine Destillationskolonne, soweit nicht im Einzelfall ein anderes thermisches Trennverfahren angegeben ist

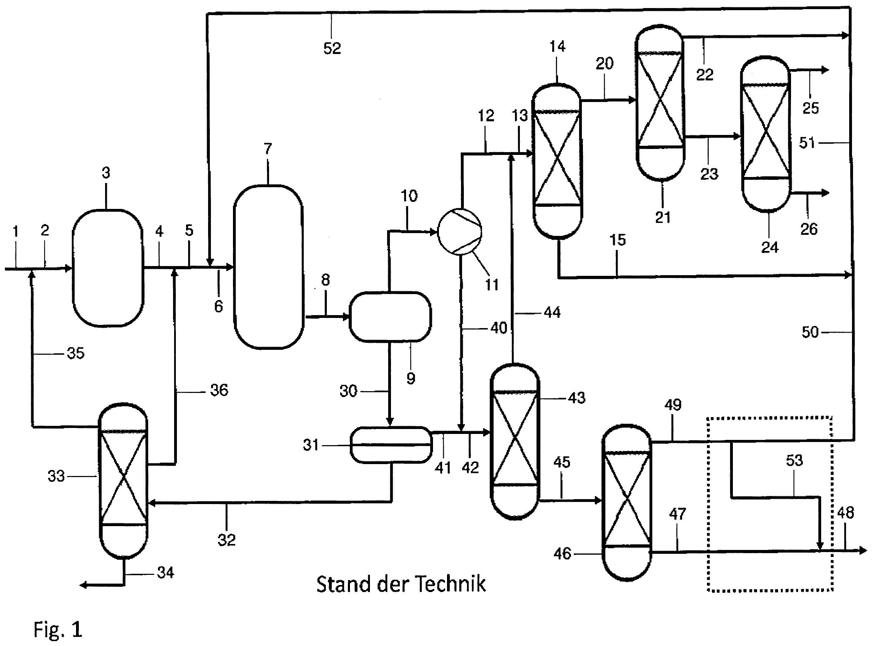

In Fig. 1 wird ein MTP-Verfahren gemäß dem Stand der Technik dargestellt. Über die Leitung 1 und 2 wird Methanol in einen Reaktor 3 eingebracht, in dem das Methanol wenigstens teilweise zu Dimethylether umgesetzt wird. Über Leitungen 4, 5 und 6 wird der Dimethylether abgezogen und einem zweiten Reaktor 7, dem Olefinsynthesereaktor, zugeführt, in dem der Dimethylether zusammen Dampf zu Olefinen umgesetzt wird. Der so erhaltene Olefinstrom enthält Propylen und andere Olefine, aber auch Paraffine, cyclische Olefine, cyclische Paraffine und Aromaten.

Der erhaltene Produktstrom wird über Leitung 8 in die Kühlvorrichtung 9 eingebracht. Dort trennt sich eine gasförmige von einer flüssigen Phase. Die gasförmige Phase enthält die C₅₋-Fraktion und wird über Leitung 10 einem Kompressor 11 zugeführt. Die in dem Kompressor 11 entstehende gasförmige Fraktion wird über die Leitungen 12 und 13 einer Destillationskolonne 14 zugeführt. In dieser Destillationskolonne 14 wird die C₃₋-Fraktion von der C₄₊-Fraktion getrennt.

Die C₃₋-Fraktion wird über Leitung 20 einer Kolonne 21 zugeführt, in der die C₂₋-Fraktion über Kopf abgezogen wird. Die C₂₋-Fraktion gelangt über Leitung 22 und Leitung 52 zurück in Leitung 5 und kann von dort über Leitung 6 in den Reaktor 7 geführt werden, so dass hier durch Olefin-Metathese zumindest teilweise das gewünschte Produkt Propylen erzeugt wird. Um eine Anreicherung von inerten leichten gasförmigen Komponenten wie Methan oder Kohlenoxiden im Kreislauf zu vermeiden, kann eine kleine Teilmenge des Stroms aus Leitung 22 über eine nicht gezeigte Leitung als Spülstrom (Purge) aus dem System entfernt werden. Weiterhin wird aus der Kolonne 21 über Leitung 23 die C₃-Fraktion abgezogen und einer Kolonne 24 zugeführt. In dieser Kolonne 24 wird über Kopf das erwünschte Zielprodukt Propylen abdestilliert und über Leitung 25 abgezogen, während im Sumpf Propan verbleibt und über Leitung 26 abgezogen wird..

Das Sumpfprodukt der Kolonne 14 wird als C₄-Fraktion über Leitung 15 aus der Kolonne 14 abgezogen und über die Leitung 51 und 52 ebenfalls vor die Umsetzung des Ethers zu Olefinen in Leitung 5 zurückgeführt, um durch Olefin-Metathese die Ausbeute an Propylen weiter zu steigern. Um eine Anreicherung von Butan (einer für die Umsetzung im Reaktor inerten Komponente) im Kreislauf zu vermeiden, kann eine kleine Teilmenge des Stroms aus Leitung 15 über eine nicht gezeigte Leitung als Purge aus dem System entfernt werden.

Die in dem Kühler 9 erhaltene flüssige Fraktion wird über Leitung 30 einem Separator 31 zugeführt. Die in dem Separator 31 abgetrennte wässrige Phase enthält auch Oxygenate (bei der Verwendung von Methanol als Edukt vor allem Methanol, aber auch Dimethylether) und wird über Leitung 32 einer Destillationskolonne 33 zugeführt.

Aus dem Sumpf der Kolonne 33 wird über Leitung 34 Wasser ausgeschleust. Weiterhin wird über Leitung 36 Dampf aus der Kolonne 33 abgezogen und in Leitung 4 eingespeist, von wo der Dampf über Leitung 5 und Leitung 6 in den Reaktor 7 gelangt, wo er als Edukt für die Umsetzung des Dimethylethers zu Olefinen verwendet wird.

Das Kopfprodukt der Kolonne 33, wenigstens ein Oxygenat, vorzugsweise Methanol, wird über Leitung 35 in die Leitung 1 eingespeist und gelangt so über Leitung 2 in den Reaktor 3. Die Kolonne 33 kann auch als Kombination mehrerer hintereinander geschalter Kolonnen ausgestaltet werden (nicht gezeigt). Wird als Edukt Methanol verwendet, so wird so rückgewonnenes Methanol zusammen mit dem als Edukt eingespeisten Methanol zu Dimethylether umgesetzt. Alternativ kann das Oxygenat auch direkt mit dem Wasserdampf über Leitung 36 zurück in den Reaktor 7 geleitet werden. Dies empfiehlt sich insbesondere dann, wenn als Oxygenat neben Methanol auch signifikante Mengen an Dimethylether vorhanden sind.

Die aus dem Separator abgezogene organische Phase enthält die C₅₊-Fraktion, welche über Leitung 41 ausgeschleust und über eine Pumpe (nicht gezeigt) weitergeleitet wird. Dieser C₅₊-Fraktion wird dann auch die aus dem Kompressor 11 bei 15 bis 25 bar gewonnene flüssige Fraktion über Leitung 40 zugemischt. Die so vereinigten Ströme werden über Leitung 42 in eine Kolonne 43 eingebracht. Über Kopf wird aus der Kolonne 43 über Leitung 44 die C₄₋-Fraktion in die Leitung 12 geführt, von wo sie zusammen mit dem gasförmigen Teil aus dem Kompressor 11 über Leitung 13 in die Kolonne 14 gespeist wird.

Über Leitung 45 wird das Sumpfprodukt der Kolonne 43, das die C₅₊-Fraktion enthält, in die Kolonne 46 geführt. Aus der Kolonne 46 wird über den Sumpf die C₇₊-Fraktion in die Leitung 47 und 48 abgezogen.

Über Kopf der Kolonne 46 wird über die Leitungen 49, 50, 51 und 52 die erhaltene C₇₋-Fraktion, die auch C₅/C₆-Kohlenwasserstoffe umfasst, recycelt, indem sie in die Leitung 5 zurückgeführt wird. Teile der C₅- und C₆-Fraktion werden über Leitung 53 der Leitung 47 zugeführt und über Leitung 48 aus dem Prozess ausgeschleust (Purge). Der aus Leitung 48 den Prozess verlassene Strom stellt das MTP-Benzin dar.

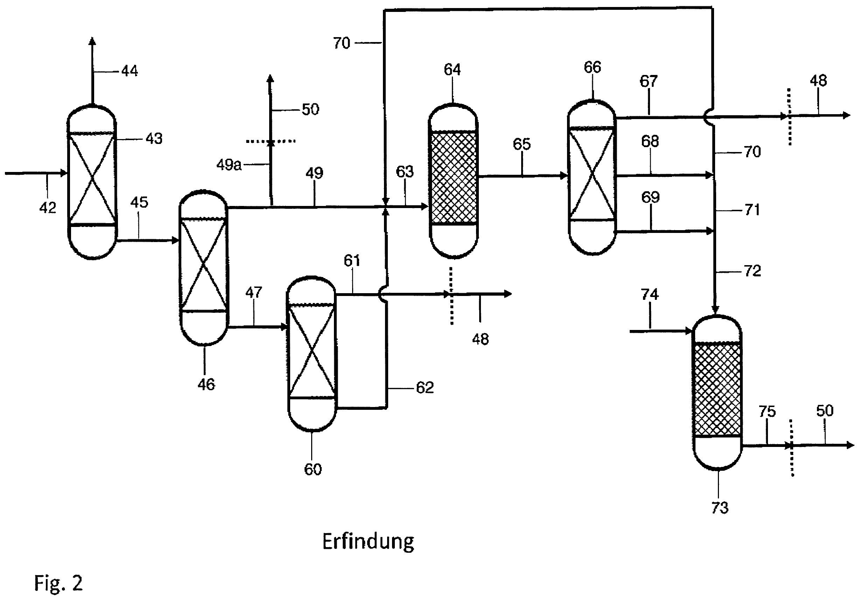

In Fig. 2 ist schematisch der Ablauf des erfindungsgemäßen Verfahrens bzw. der Aufbau einer erfindungsgemäßen Anlage dargestellt. Das erfindungsgemäße Verfahren bzw. die erfindungsgemäßen Anlage entspricht bis zu der Anlagenkomponente 52 dem bereits aus dem Stand der Technik bekannten Verfahren. Die Anordnung des im Rahmen der Erfindung modifizierten Teils zwischen den Leitungen 47, 48, 49 und 50 ist in Fig. 1 durch einen gepunkteten Rahmen angedeutet. Korrespondierend dazu bedeuten in Fig. 2 die gepunkteten Linien Anbindungsstellen an das in Fig. 1 gezeigte Grundverfahren. Leitung 53 entfällt im erfindungsgemäßen Verfahren bzw. bei der erfindungsgemäßen Anlage.

Über Leitung 47 wird die C₇₊-Fraktion in eine Kolonne 60 eingebracht In einer vorteilhaften Ausgestaltung wird diese Kolonne 60 als Extraktivdestillation betrieben und ihr ein zusätzlicher Strom zugeführt, der vorteilhafte chemische und physikalische Eigenschaften aufweist, so dass eine noch bessere Trennung zwischen C₇₊-Olefinen und - Paraffinen im Kopf und Aromaten im Sumpf möglich ist. Bei dem als Extraktivmittel verwendeten Strom kann es sich beispielsweise um ein Olefin bzw. einen aromatenreichen Strom handeln, der bevorzugt innerhalb der Anlage erzeugt und rezirkuliert wird. Es können aber auch externe Hilfsstoffe wie beispielsweise N-Methyl-pyrrolidon (NMP) eingesetzt werden. Das Funktionsprinzip beruht darauf, dass sich entweder die Olefine im Kopf oder die Aromaten im Sumpf anreichern.

Am Kopf der Kolonne 60 wird über Leitung 61 eine Fraktion abgeführt, die die C₇₊-Olefine und -Paraffine enthält. Diese Fraktion wird über Leitung 48 dem MTP-Benzin zugeschlagen.

Die im Sumpf der Kolonne 60 gewonnene Aromatenfraktion wird über Leitung 62 und 63 dem Alkylierungsreaktor 64 zugeführt, der als adiabater Festbettreaktor ausgestaltet und in diesem Ausführungsbeispiel mit demselben Katalysator gefüllt ist wie der Olefinsynthesereaktor, einem handelsüblichen Zeolith-Katalysator des Strukturtyps ZSM-5. Es können aber auch andere geeignete Alkylierungskatalysatoren verwendet werden, die der Handel bereitstellt. Dem Alkylierungsreaktor wird ferner auch die am Kopf der Kolonne 46 gewonnene C₇₋-Fraktion zugeführt, die auch C₅/C₆-Olefine umfasst. Im Alkylierungsreaktor erfolgt die Umsetzung der C₅/C₆-Olefine mit den Aromaten bei Temperaturen zwischen 80 und 180 °C. Der Druck wird dabei so hoch gewählt, dass die Umsetzung in der Flüssigphase erfolgt; er liegt zwischen 1 und 20 bar, absolut. Als Alkylierungsprodukte werden dabei Aromaten mit C₅- bzw. C₆-Seitenketten gebildet, wobei je nach Art des Ausgangsaromaten der aromatische Kern entweder keine weitere Alkylgruppe (Benzol), eine weitere Alkylgruppe (Toluol) oder mehrere weitere Alkylgruppen (Xylole etc.) trägt. Mehrfachalkylierungen desselben aromatischen Kerns durch mehrere Olefinmoleküle sind unerwünscht, da die entstehenden Moleküle zu sperrig sind und im Olefinsynthesereaktor nicht mehr gut umgesetzt werden können, da sie nur schwer in die Poren des dort verwendeten Katalysators diffundieren können. Die Bildung mehrfach alkylierter Produkte wird durch die Einstellung eines Stoffmengenverhältnisses der Aromaten in Leitung 63 zu den Olefinen in derselben Leitung von >1 mol/mol weiter unterdrückt. Zudem wird ihre Bildung durch die Formselektivität des auch als Alkylierungskatalysator verwendeten ZSM-5-Zeoliths reduziert.

Über Leitung 65 wird das Produkt des Alkylierungsreaktors, das neben Alkylaromaten auch nicht umgesetzte Ausgangsaromaten und nicht umgesetzte Kohlenwasserstoffe, insbesondere Paraffine, umfasst, der Kolonne 66 zugeführt. In der Kolonne 66, die auch mehrere hintereinander geschaltete Kolonnen umfassen kann, erfolgt die destillative Auftrennung in eine niedrig siedende Fraktion, die C₅/C₆-Kohlenwasserstoffe, insbesondere die C₅/C₆-Paraffine umfasst und über Leitung 67 und 48 dem MTP-Benzin zugeschlagen wird, ferner in eine im mittleren Temperaturbereich siedende Fraktion, die vor allem nicht umgesetzte Ausgangsaromaten umfasst und zum Teil über Leitung 68, 70 und 63 zum Alkylierungsreaktor 64 zurückgeführt und zum anderen Teil über Leitung 68, 71 und 72 zum Hydrierungsreaktor 73 geführt wird, und schließlich eine schwersiedende Fraktion, die Alkylaromaten umfasst und über Leitung 69 und 72 ebenfalls zu dem Hydrierungsreaktor 73 geführt wird.

Der Hydrierungsreaktor 73 kann z. B. ein baulich einfacher Festbettreaktor sein, es können aber auch Reaktoren mit interner Kühlung, ein oder mehrstufig, verwendet werden. Als Katalysatoren können beispielsweise Nickel, Palladium, Platin, weitere Edelmetalle oder deren Mischungen auf Trägermaterialien wie Aktivkohle, Siliciumoxid oder Aluminiumoxid verwendet werden.

Im Hydrierungsreaktor 73 erfolgt die Hydrierung aller zugeführten Aromaten zu den entsprechenden Cycloparaffinen mit Wasserstoff, er über Leitung 74 zugeführt wird, bei Temperaturen zwischen 100 und 250 °C und Drücken zwischen 1 und 40 bar, absolut. Am Ausgang des Hydrierungsreaktors wird nicht umgesetzter Wasserstoff auf an sich bekannte Weise abgetrennt und mittels einer Rückführleitung (nicht gezeigt) zum Reaktoreingang des Hydrierungsreaktors zurückgeführt. Da dieser Wasserstoffstrom auch gebildete leichte Gase wie Methan enthält, muss auch an dieser Stelle wiederum ein Teilstrom aus dem Kreislauf über eine nicht gezeigte Leitung (Purge) entfernt werden.

Ferner wird ein Teil des Hydrierungsproduktes über eine weitere, nicht gezeigte Rückführleitung zum Reaktoreingang des Hydrierungsreaktors zurückgeführt. Auf diese Weise wird der Temperaturanstieg im Hydrierungsreaktor aufgrund der stark exothermen Hydrierungsreaktionen wirkungsvoll begrenzt. Das Verhältnis zwischen dem zurückgeführten hydrierten Produkt und den nicht-hydrierten Alkylaromaten liegt dabei zwischen 1 kg/kg und 10 kg/kg. Der molare Überschuss von Wasserstoff bei der Hydrierung liegt aufgrund der Rückführung zwischen 200 und 5000 % der theoretisch für die vollständige Absättigung aller enthaltener Doppel- und aromatischen Bindungen benötigten Menge.

Um eine vorteilhafte Ausgestaltung des Prozesses in energetischer Hinsicht sicherzustellen, wird der Hydrierreaktor 73 bei annähernd gleicher Temperatur wie der Sumpf der Kolonne 66 gefahren, wodurch auf einen sonst notwendigen Wärmetauscher zwischen diesen beiden Anlagenteilen verzichtet werden kann, wodurch sich die Investitions- und Betriebskosten des Prozesses reduzieren und sich die Wirtschaftlichkeit verbessert.

Über Leitung 75 wird der nicht zurückgeführte Anteil des Hydrierungsproduktes aus dem Hydrierungsreaktor abgeführt und über Leitung 50, 51, 52 und 6 zum Olefinsynthesereaktor 7 zurückgeführt, in dem die mindestens teilweise Umsetzung der Cycloparaffine zu Olefinen, insbesondere zu Propylen, erfolgt.

Das in Fig. 2 dargestellte Verfahren hat den Vorteil, dass das in Fig. 1 dargestellte Kernsystem eines üblichen MTP-Verfahrens identisch bleibt und im Wesentlichen nicht neu ingenieurstechnisch ausgelegt werden muss. Ferner werden insbesondere die C₅/C₆-Olefine und auch die Aromaten besser für die Umwandlung zu Propylen genutzt, sie gehen nicht, wie in dem Verfahren nach Fig. 1, über das MTP-Benzin bzw. einen Spülstrom verloren. Als Ergebnis wird die Menge des ursprünglichen MTP-Benzins deutlich reduziert und die Propylen-Ausbeute des Gesamtverfahrens gesteigert.

### Zahlenbeispiel

Bei Simulationsrechnungen, die den MTP-Prozess nach dem Stand der Technik abbilden, wurde für den Stoffstrom in Leitung 49 folgende Zusammensetzung ermittelt:

**Leitung 49:**

| | |
|---|---|
| C₅-Olefine: | 4,0 g/h |
| C₆-Olefine: | 3,1 g/h |
| Summe | 7,1 g/h |

Gleichzeitig wurde für den Stoffstrom in Leitung 47 folgende Zusammensetzung ermittelt:

**Leitung 47:**

| | |
|---|---|
| Toluol: | 3,0 g/h |
| Xylole: | 15,0 g/h |
| Summe | 18,0 g/h |

Der Propylen-Produkt-Strom (entsprechend Leitung 25) war 258,8 g/h.

Gemäß Stand der Technik werden die Olefine in Leitung 49 über den Purge ungenutzt dem Prozess entzogen; durch die erfindungsgemäße Alkylierung können sie dagegen wiedergewonnen und zurückgeführt werden. Im obigen Beispiel enthält der Stoffstrom in Leitung 49 94 mmol Olefine und der Stoffstrom in Leitung 47 174 mmol Aromaten, was einem Molverhältnis von 1,85 entspricht.

Der Alkylierungsreaktor 63 wird demnach über Strom 47 und 49 mit 25,1 g/h Feed beschickt. Der Paraffinanteil wird nicht berücksichtigt, da sich die Paraffine inert verhalten.

Unter der Annahme, dass 80% zielführend alkyliert werden, ergibt sich ein Alkylierungsprodukt (Leitung 69) von 13,7 g/h sowie nicht umgesetzte Aromaten (Strom 68) von 10,0 g/h. Die nicht umgesetzten Olefine (1,4 g/h) werden der Anlage weiterhin über Leitung 70 entzogen.

Über Leitung 72 wird dann ein Strom von 23,7 g/h dem Hydrierreaktor 73 zugeführt, dem dann nach vollständiger Hydrierung von Alkylat und Aromaten ein hydriertes Produkt (Strom 75) von 24,7 g/h über Leitung 50 in den Reaktor 7 zurückgeschickt.

In vereinfachter Weise kann man davon ausgehen, dass unter Reaktionsbedingungen die Alkylierungsprodukte wieder gespalten werden, wobei Olefine (ca. 5,7 g/h) und Cyclohexan-Derivate entstehen (ca. 19,1 g/h) entstehen. Durch Umsetzung dieser Komponenten im Reaktor 7 entstehen ca. 8,5 g/h mehr Propylen (verglichen mit 258,8 g/h für den Prozess nach Stand der Technik).

Durch die Umsetzung dieser Ströme zu Alkylaromaten und ihre Rückführung zum Olefinsynthesereaktor konnte die Propylenausbeute um rund 3 % gesteigert werden.

### Gewerbliche Anwendbarkeit

Mit der Erfindung wird eine Verbesserung des MTP-Verfahrens zur Verfügung gestellt, die es ermöglicht, die ansonsten dem Benzinprodukt zugeschlagenen oder über Spülströme dem Verfahren entzogenen C₅/C₆-Olefine und einen Teil der aromatischen Produkte zumindest teilweise in das Zielprodukt Propylen umzuwandeln. Gleichzeitig wird die Menge des als Koppelprodukt anfallenden MTP-Benzins reduziert, dessen Weiterverarbeitung und Vermarktung unter Umständen aufwendig ist.

### Bezugszeichenliste

- 1, 2: Leitung
- 3: DME-Reaktor
- 4 - 6: Leitung
- 7: Olefinsynthesereaktor
- 8: Leitung
- 9: Kühler
- 10: Leitung
- 11: Kompressor
- 12, 13: Leitung
- 14: Destillationskolonne
- 15: Leitung
- 20: Leitung
- 21: Destillationskolonne
- 22, 23: Leitung
- 24: Destillationskolonne
- 25, 26: Leitung
- 30: Leitung
- 31: Separator
- 32: Leitung
- 33: Kolonne
- 34 - 36: Leitung
- 40 - 42: Leitung
- 43: Destillationskolonne
- 44, 45: Leitung
- 46: Destillationskolonne
- 47 - 53: Leitung
- 49a: Leitung
- 60: Extraktivdestillationskolonne
- 61 - 63: Leitung
- 64: Alkylierungsreaktor
- 65: Leitung
- 66: Destillationskolonne
- 67 - 72: Leitung
- 73: Hydrierungsreaktor
- 74, 75: Leitung

## Patentansprüche

1. Verfahren zur Herstellung von Olefinen, insbesondere Propylen, aus Oxygenaten, umfassend die folgenden Schritte:
(a) heterogen-katalysierte Umsetzung eines Wasserdampf und Oxygenate, wie Methanol und/oder Dimethylether, umfassenden Eduktgemischs unter Oxygenatumsetzungsbedingungen in einem Olefinsynthesereaktor zu einem Propylen, andere Olefine, Paraffine und Aromaten enthaltenden Primärprodukt,
(b) Auftrennung des Primärprodukts mittels physikalischer Trennverfahren in eine C₅₋-Fraktion, eine C₅₊-Fraktion und eine wässrige, nicht umgesetzte Oxygenate enthaltende Phase,
(c) Auftrennung der C₅₋-Fraktion durch mehrstufige Destillation, wobei ein Propylenproduktstrom und mehrere olefinhaltige Kohlenwasserstofffraktionen erhalten werden, wobei letztere mindestens teilweise zum Olefinsynthesereaktor zurückgeführt werden,
(d) Abtrennung der nicht umgesetzten Oxygenate aus der wässrigen Phase mittels eines thermischen Trennverfahrens, Rückführung der abgetrennten, nicht umgesetzten Oxygenate und mindestens eines Teils des Wassers zum Olefinsynthesereaktor,
(e) Auftrennung der C₅₊-Fraktion durch mehrstufige Destillation, wobei eine C₇₋-Fraktion erhalten wird, die C₅/C₆-Olefine umfasst, und wobei eine C₇₊-Fraktion erhalten wird, die Aromaten umfasst,
(f) Auftrennung der C₇₊-Fraktion durch ein thermisches Trennverfahren, bevorzugt durch Extraktivdestillation, in eine Aromatenfraktion und eine die C₇₊-Paraffine umfassende Fraktion,
(g) Zuführung mindestens eines Teils der die C₅/C₆-Olefine umfassenden C₇₋-Fraktion und mindestens eines Teils der Aromatenfraktion zu einem Alkylierungsreaktor, in dem durch heterogen-katalysierte Umsetzung der Aromaten mit den C₅/C₆-Olefinen an einem Alkylierungskatalysator unter Alkylierungsbedingungen ein Alkylierungsprodukt erhalten wird, das Alkylaromaten umfasst,
(h) Auftrennung des Alkylierungsprodukts durch ein thermisches Trennverfahren in einen C₅/C₆-Paraffine enthaltenden Strom, einen Alkylaromatenstrom und einen nicht umgesetzte Aromaten enthaltenden Strom, der mindestens teilweise zum Alkylierungsreaktor zurückgeführt wird,
(i) Zuführung des Alkylaromatenstroms zu einem Hydrierungsreaktor, in dem durch heterogen-katalysierte Umsetzung der Alkylaromaten an einem Hydrierungskatalysator unter Hydrierungsbedingungen ein Hydrierungsprodukt erhalten wird, das Cycloparaffine umfasst,
(j) Rückführung mindestens eines Teils des Hydrierungsprodukts zum Olefinsynthesereaktor.

2. Verfahren nach Anspruch 1, **dadurch gekennzeichnet, dass** in der Hydrierung Wasserstoff als Hydrierungsmittel verwendet wird.

3. Verfahren nach Anspruch 1 oder 2, **dadurch gekennzeichnet, dass** die Hydrierung so durchgeführt wird, dass Teile des Produktstroms der Hydrierung in die Hydrierung zurückgeführt werden und dass das Verhältnis zwischen dem zurückgeführten hydrierten Produkt und nicht-hydrierten Alkylaromaten zwischen 1 kg/kg und 10 kg/kg liegt.

4. Verfahren nach einem der vorhergehenden Ansprüche, **dadurch gekennzeichnet, dass** nach Schritt (i) Wasserstoff abgetrennt und zum Hydrierungsreaktor zurückgeführt wird, wobei der molare Überschuss von Wasserstoff bei der Hydrierung zwischen 200 und 5000 % der theoretisch für die vollständige Absättigung aller enthaltener Doppel- und aromatischen Bindungen benötigten Menge liegt.

5. Verfahren nach einem der vorhergehenden Ansprüche, **dadurch gekennzeichnet, dass** Verfahrensschritt (a) in zwei Stufen erfolgt, wobei in der ersten Stufe Methanol in Dimethylether umgewandelt und in der zweiten Stufe Dimethylether zu einem Propylen, andere Olefine, Paraffine und Aromaten enthaltenden Primärprodukt umgesetzt wird und wobei Methanol vor die erste Stufe und/oder Wasser dampfförmig vor die zweite Stufe zurückgeführt wird/werden.

6. Verfahren nach einem der vorhergehenden Ansprüche, **dadurch gekennzeichnet, dass** mindestens ein Teil der in Verfahrensschritt (e) erhaltenen, die C₅/C₆-Olefine umfassenden C₇₋-Fraktion zum Olefinsynthesereaktor zurückgeführt wird.

7. Verfahren nach einem der vorhergehenden Ansprüche, **dadurch gekennzeichnet, dass** mindestens ein Teil des in Verfahrensschritt (h) erhaltenen, nicht umgesetzte Aromaten enthaltenden Stroms dem Hydrierungsreaktor zugeführt wird.

8. Verfahren nach einem der vorhergehenden Ansprüche, **dadurch gekennzeichnet, dass** in dem Olefinsynthesereaktor und in dem Alkylierungsreaktor derselbe Katalysator verwendet wird.

9. Verfahren nach einem der vorhergehenden Ansprüche, **dadurch gekennzeichnet, dass** im Verfahrensschritt (g) das Verhältnis der Summe der Mole von Aromaten in den aromatenhaltigen Strömen bzw. Fraktionen zur Summe der Mole an Olefinen in der dem Alkylierungsreaktor zugeführten C₇₋-Fraktion zwischen 1 und 10 mol/mol, bevorzugt zwischen 1 und 5 mol/mol, meist bevorzugt zwischen 1 und 2 mol/mol liegt.

10. Anlage zur Herstellung von Olefinen aus Oxygenaten, insbesondere zur Durchführung des Verfahrens nach einem der vorhergehenden Ansprüche, umfassend folgende miteinander in Fluidverbindung stehenden Anlagenteile:
- wenigstens einen Olefinsynthesereaktor zur heterogen-katalysierten Umsetzung von wenigstens einem Oxygenat zu Propylen, andere Olefine, Paraffine und Aromaten enthaltenden Primärprodukt,
- eine Trennvorrichtung zur Auftrennung des Primärprodukts in eine C₅₋-Fraktion, eine C₅₊-Fraktion und eine wässrige, nicht umgesetzte Oxygenate enthaltende Phase,
- eine Trennvorrichtung zur Auftrennung der C₅₋-Fraktion in einen Propylenproduktstrom und mehrere olefinhaltige Kohlenwasserstofffraktionen, sowie Rückführleitungen zur Rückführung mindestens eines Teils der olefinhaltigen Kohlenwasserstofffraktionen zum Olefinsynthesereaktor,
- eine Trennvorrichtung zur Abtrennung der nicht umgesetzten Oxygenate aus der wässrigen Phase, sowie Rückführleitungen zur Rückführung mindestens eines Teils der nicht umgesetzten Oxygenate und mindestens eines Teils des Wassers zum Olefinsynthesereaktor,
- eine Trennvorrichtung zur Auftrennung der C₅₊-Fraktion in eine C₇₋-Fraktion, die C₅/C₆-Olefine umfasst, und in eine C₇₊-Fraktion, die Aromaten umfasst,
- eine Trennvorrichtung zur Auftrennung der C₇₊-Fraktion in eine Aromatenfraktion und eine die C₇₊-Paraffine umfassende Fraktion,
- einen Alkylierungsreaktor zur heterogen-katalysierten Umsetzung von Aromaten mit C₅/C₆-Olefinen zu einem Alkylierungsprodukt, das Alkylaromaten umfasst, sowie Leitungen für die Zuführung mindestens eines Teils der die C₅/C₆-Olefine umfassenden C₇₋-Fraktion und mindestens eines Teils der Aromatenfraktion zu dem Alkylierungsreaktor,
- eine Trennvorrichtung zur Auftrennung des Alkylierungsprodukts in einen C₅/C₆-Paraffine enthaltenden Strom, einen Alkylaromatenstrom und einen nicht umgesetzte Aromaten enthaltenden Strom, sowie eine Rückführleitung für die Rückführung mindestens eines Teils des nicht umgesetzte Aromaten enthaltenden Stroms zum Alkylierungsreaktor,
- einen Hydrierungsreaktor zur Hydrierung der Alkylaromaten zu einem Hydrierungsprodukt, das Cycloparaffine umfasst,
- eine Rückführleitung zur Rückführung mindestens eines Teils des Hydrierungsprodukts zum Olefinsynthesereaktor.

11. Anlage nach Anspruch 10, **gekennzeichnet durch** eine Rückführleitung für die Rückführung mindestens eines Teils der die C₅/C₆-Olefine umfassenden C₇₋-Fraktion zum Olefinsynthesereaktor.

12. Anlage nach Anspruch 10 oder 11, **gekennzeichnet durch** eine Leitung zur Zuführung mindestens eines Teils des die nicht umgesetzten Aromaten enthaltenden Stroms zum Hydrierungsreaktor.

## Claims

1. A process for producing olefins, in particular propylene, from oxygenates, comprising the following steps:
(a) heterogeneously catalyzed conversion of an educt mixture comprising steam and oxygenates, such as methanol and/or dimethyl ether, under oxygenate conversion conditions in an olefin synthesis reactor to a primary product containing propylene, other olefins, paraffins and aromatics,
(b) separation of the primary product by means of physical separation processes into a C₅₋ fraction, a C₅₊ fraction and an aqueous phase containing non-converted oxygenates,
(c) separation of the C₅₋ fraction by multistage distillation, wherein a propylene product stream and several olefin-containing hydrocarbon fractions are obtained, wherein the latter are at least partly recirculated to the olefin synthesis reactor,
(d) separation of the non-converted oxygenates from the aqueous phase by means of a thermal separation process, recirculation of the separated, non-converted oxygenates and at least a part of the water to the olefin synthesis reactor,
(e) separation of the C₅₊ fraction by multistage distillation, wherein a C₇₋ fraction is obtained which comprises C₅/C₆ olefins, and wherein a C₇₊ fraction is obtained which comprises aromatics,
(f) separation of the C₇₊ fraction by a thermal separation process, preferably by extractive distillation into an aromatics fraction and a fraction comprising the C₇₊ paraffins,
(g) supplying at least a part of the C₇₋ fraction comprising the C₅/C₆ olefins and at least a part of the aromatics fraction to an alkylation reactor in which by heterogeneously catalyzed conversion of the aromatics with the C₅/C₆ olefins on an alkylation catalyst under alkylation conditions an alkylation product is obtained, which comprises alkyl aromatics,
(h) separation of the alkylation product by a thermal separation process in a stream containing C₅/C₆ paraffins, an alkyl aromatics stream and a stream containing non-converted aromatics, which is at least partly recirculated to the alkylation reactor,
(i) supplying the alkyl aromatics stream to a hydrogenation reactor in which by heterogeneously catalyzed conversion of the alkyl aromatics on a hydrogenation catalyst under hydrogenation conditions a hydrogenation product is obtained, which comprises cycloparaffins,
(j) recirculation of at least a part of the hydrogenation product to the olefin synthesis reactor.

2. The process according to claim 1, **characterized in that** in the hydrogenation hydrogen is used as hydrogenating agent.

3. The process according to claim 1 or 2, **characterized in that** the hydrogenation is carried out such that parts of the product stream of the hydrogenation are recirculated into the hydrogenation, and that the ratio between the recirculated hydrogenated product and non-hydrogenated alkyl aromatics lies between 1 kg/kg and 10 kg/kg.

4. The process according to any of the preceding claims, **characterized in that** after step (i) hydrogen is separated and recirculated to the hydrogenation reactor, wherein the molar excess of hydrogen during the hydrogenation lies between 200 and 5000 % of the quantity theoretically necessary for the complete saturation of all contained double and aromatic bonds.

5. The process according to any of the preceding claims, **characterized in that** process step (a) is effected in two stages, wherein in the first stage methanol is converted into dimethyl ether and in the second stage dimethyl ether is converted to a primary product containing propylene, other olefins, paraffins and aromatics, and wherein methanol is recirculated to before the first stage and/or water is recirculated in the form of steam to before the second stage.

6. The process according to any of the preceding claims, **characterized in that** at least a part of the C₇₋ fraction obtained in process step (e), which comprises the C₅/C₆ olefins, is recirculated to the olefin synthesis reactor.

7. The process according to any of the preceding claims, **characterized in that** at least a part of the stream obtained in process step (h), which contains non-converted aromatics, is supplied to the hydrogenation reactor.

8. The process according to any of the preceding claims, **characterized in that** in the olefin synthesis reactor and in the alkylation reactor the same catalyst is used.

9. The process according to any of the preceding claims, **characterized in that** in process step (g) the ratio of the sum of the moles of aromatics in the aromatics-containing streams or fractions to the sum of the moles of olefins in the C₇₋ fraction supplied to the alkylation reactor lies between 1 and 10 mol/mol, preferably between 1 and 5 mol/mol, most preferably between 1 and 2 mol/mol.

10. A plant for producing olefins from oxygenates, in particular for carrying out the process according to any of the preceding claims, comprising the following plant sections which are in fluid connection with each other:
- at least one olefin synthesis reactor for the heterogeneously catalyzed conversion of at least one oxygenate to a primary product containing propylene, other olefins, paraffins and aromatics,
- a separating device for separating the primary product into a C₅₋ fraction, a C₅₊ fraction and an aqueous phase containing non-converted oxygenates,
- a separating device for separating the C₅₋ fraction into a propylene product stream and several olefin-containing hydrocarbon fractions, as well as return conduits for recirculating at least a part of the olefin-containing hydrocarbon fractions to the olefin synthesis reactor,
- a separating device for separating the non-converted oxygenates from the aqueous phase, as well as return conduits for recirculating at least a part of the non-converted oxygenates and at least a part of the water to the olefin synthesis reactor,
- a separating device for separating the C₅₊ fraction into a C₇₋ fraction which comprises C₅/C₆ olefins, and into a C₇₊ fraction which comprises aromatics,
- a separating device for separating the C₇₊ fraction into an aromatics fraction and a fraction comprising the C₇₊ paraffins,
- an alkylation reactor for the heterogeneously catalyzed conversion of aromatics with C₅/C₆ olefins to an alkylation product which comprises alkyl aromatics, as well as conduits for supplying at least a part of the C₇₋ fraction comprising the C₅/C₆ olefins and at least a part of the aromatics fraction to the alkylation reactor,
- a separating device for separating the alkylation product into a stream containing C_{5/}C₆ paraffins, an alkyl aromatics stream and a stream containing non-converted aromatics, as well as a return conduit for recirculating at least a part of the stream containing non-converted aromatics to the alkylation reactor,
- a hydrogenation reactor for hydrogenating the alkyl aromatics to a hydrogenation product which comprises cycloparaffins,
- a return conduit for recirculating at least a part of the hydrogenation product to the olefin synthesis reactor.

11. The plant according to claim 10, **characterized by** a return conduit for the recirculation of at least a part of the C₇₋ fraction comprising the C₅/C₆ olefins to the olefin synthesis reactor.

12. The plant according to claim 10 or 11, **characterized by** a conduit for supplying at least a part of the stream containing the non-converted aromatics to the hydrogenation reactor.

## Revendications

1. Procédé de fabrication d'oléfines, notamment de propylène, à partir de composés oxygénés, comprenant les étapes suivantes :
(a) la mise en réaction sous catalyse hétérogène d'un mélange de réactifs comprenant de la vapeur d'eau et des composés oxygénés, tels que du méthanol et/ou de l'éther diméthylique, dans des conditions de réaction de composés oxygénés dans un réacteur de synthèse d'oléfines pour former un produit primaire contenant du propylène, d'autres oléfines, des paraffines et des composés aromatiques,
(b) la séparation du produit primaire au moyen de procédés de séparation physiques en une fraction en C₅₋, une fraction en C₅₊ et une phase aqueuse contenant des composés oxygénés non réagis,
(c) la séparation de la fraction en C₅₋ par distillation à plusieurs étapes, un courant de produit de propylène et plusieurs fractions hydrocarbonées contenant des oléfines étant obtenus, ces dernières étant au moins partiellement recyclées dans le réacteur de synthèse d'oléfines,
(d) la séparation des composés oxygénés non réagis de la phase aqueuse au moyen d'un procédé de séparation thermique, le recyclage des composés oxygénés non réagis séparés et d'au moins une partie de l'eau dans le réacteur de synthèse d'oléfines,
(e) la séparation de la fraction en C₅₊ par distillation à plusieurs étapes, une fraction en C₇₋ étant obtenue, qui comprend des oléfines en C₅/C₆, et une fraction en C₇₊ étant obtenue, qui comprend des composés aromatiques,
(f) la séparation de la fraction en C₇₊ par un procédé de séparation thermique, de préférence par distillation extractive, en une fraction de composés aromatiques et une fraction comprenant les paraffines en C₇₊,
(g) l'introduction d'au moins une partie de la fraction en C₇₋ comprenant les oléfines en C₅/C₆ et d'au moins une partie de la fraction de composés aromatiques dans un réacteur d'alkylation, dans lequel un produit d'alkylation qui comprend des composés alkylaromatiques est obtenu par mise en réaction sous catalyse hétérogène des composés aromatiques avec les oléfines en C₅/C₆ sur un catalyseur d'alkylation en conditions d'alkylation,
(h) la séparation du produit d'alkylation par un procédé de séparation thermique en un courant contenant des paraffines en C₅/C₆, un courant de composés alkylaromatiques et un courant contenant des composés aromatiques non réagis, qui est au moins partiellement recyclé dans le réacteur d'alkylation,
(i) l'introduction du courant de composés alkylaromatiques dans un réacteur d'hydrogénation, dans lequel un produit d'hydrogénation qui comprend des cycloparaffines est obtenu par mise en réaction sous catalyse hétérogène des composés alkylaromatiques sur un catalyseur d'hydrogénation dans des conditions d'hydrogénation,
(j) le recyclage d'au moins une partie du produit d'hydrogénation dans le réacteur de synthèse d'oléfines.

2. Procédé selon la revendication 1, **caractérisé en ce que** de l'hydrogène est utilisé en tant qu'agent d'hydrogénation dans l'hydrogénation.

3. Procédé selon la revendication 1 ou 2, **caractérisé en ce que** l'hydrogénation est réalisée de telle sorte que des parties du courant de produit de l'hydrogénation sont recyclées dans l'hydrogénation et que le rapport entre le produit hydrogéné recyclé et les composés alkylaromatiques non hydrogénés est compris entre 1 kg/kg et 10 kg/kg.

4. Procédé selon l'une quelconque des revendications précédentes, **caractérisé en ce que** l'hydrogène est séparé après l'étape (i) et recyclé dans le réacteur d'hydrogénation, l'excès molaire d'hydrogène lors de l'hydrogénation étant compris entre 200 et 5 000 % de la quantité nécessaire en théorie pour la saturation totale de toutes les doubles liaisons et les liaisons aromatiques contenues.

5. Procédé selon l'une quelconque des revendications précédentes, **caractérisé en ce que** l'étape de procédé (a) a lieu en deux étapes, du méthanol étant transformé en éther diméthylique lors de la première étape et, l'éther diméthylique étant transformé en un produit primaire contenant du propylène, d'autres oléfines, des paraffines et des composés aromatiques lors de la deuxième étape, et du méthanol étant recyclé avant la première étape et/ou de l'eau étant recyclée sous forme vapeur avant la deuxième étape.

6. Procédé selon l'une quelconque des revendications précédentes, **caractérisé en ce qu'**au moins une partie de la fraction en C₇₋ comprenant des oléfines en C₅/C₆ obtenue à l'étape de procédé (e) est recyclée dans le réacteur de synthèse d'oléfines.

7. Procédé selon l'une quelconque des revendications précédentes, **caractérisé en ce qu'**au moins une partie du courant contenant des composés aromatiques non réagis obtenu à l'étape de procédé (h) est introduite dans le réacteur d'hydrogénation.

8. Procédé selon l'une quelconque des revendications précédentes, **caractérisé en ce que** le même catalyseur est utilisé dans le réacteur de synthèse d'oléfines et dans le réacteur d'alkylation.

9. Procédé selon l'une quelconque des revendications précédentes, **caractérisé en ce qu'**à l'étape de procédé (g), le rapport entre la somme des moles de composés aromatiques dans les courants ou fractions contenant des composés aromatiques et la somme des moles d'oléfines dans la fraction en C₇₋ introduite dans le réacteur d'alkylation est compris entre 1 et 10 mol/mol, de préférence entre 1 et 5 mol/mol, de manière préférée entre toutes entre 1 et 2 mol/mol.

10. Unité pour la fabrication d'oléfines à partir de composés oxygénés, notamment pour la réalisation du procédé selon l'une quelconque des revendications précédentes, comprenant les parties d'unités suivantes en connexion fluidique les unes avec les autres :
- au moins un réacteur de synthèse d'oléfines pour la mise en réaction sous catalyse hétérogène d'au moins un composé oxygéné en un produit primaire contenant du propylène, d'autres oléfines, des paraffines et des composés aromatiques,
- un dispositif de séparation pour la séparation du produit primaire en une fraction en C₅₋, une fraction en C₅₊ et une phase aqueuse contenant des composés oxygénés non réagis,
- un dispositif de séparation pour la séparation de la fraction en C₅₋ en un courant de produit de propylène et plusieurs fractions hydrocarbonées contenant des oléfines, ainsi que des conduites de recyclage pour le recyclage d'au moins une partie des fractions hydrocarbonées contenant des oléfines dans le réacteur de synthèse d'oléfines,
- un dispositif de séparation pour la séparation des composés oxygénés non réagis de la phase aqueuse, ainsi que des conduites de recyclage pour le recyclage d'au moins une partie des composés oxygénés non réagis et d'au moins une partie de l'eau dans le réacteur de synthèse d'oléfines,
- un dispositif de séparation pour la séparation de la fraction C₅₊ en une fraction en C₇₋, qui comprend des oléfines en C₅/C₆, et en une fraction en C₇₊, qui comprend des composés aromatiques,
- un dispositif de séparation pour la séparation de la fraction en C₇₊ en une fraction de composés aromatiques et une fraction comprenant les paraffines en C₇₊,
- un réacteur d'alkylation pour la mise en réaction sous catalyse hétérogène de composés aromatiques avec des oléfines en C₅/C₆ pour former un produit d'alkylation, qui comprend des composés alkylaromatiques, ainsi que des conduites pour l'introduction d'au moins une partie de la fraction en C₇₋ comprenant les oléfines en C₅/C₆ et d'au moins une partie de la fraction de composés aromatiques dans le réacteur d'alkylation,
- un dispositif de séparation pour la séparation du produit d'alkylation en un courant contenant des paraffines en C₅/C₆, un courant de composés alkylaromatiques et un courant contenant des composés aromatiques non réagis, ainsi qu'une conduite de recyclage pour le recyclage d'au moins une partie du courant contenant des composés aromatiques non réagis dans le réacteur d'alkylation,
- un réacteur d'hydrogénation pour l'hydrogénation des composés alkylaromatiques en un produit d'hydrogénation, qui comprend des cycloparaffines,
- une conduite de recyclage pour le recyclage d'au moins une partie du produit d'hydrogénation dans le réacteur de synthèse d'oléfines.

11. Unité selon la revendication 10, **caractérisée par** une conduite de recyclage pour le recyclage d'au moins une partie de la fraction en C₇₋ comprenant les oléfines en C₅/C₆ dans le réacteur de synthèse d'oléfines.

12. Unité selon la revendication 10 ou 11, **caractérisée par** une conduite pour l'introduction d'au moins une partie du courant contenant les composés aromatiques non réagis dans le réacteur d'hydrogénation.
